# EUROPEAN PATENT SPECIFICATION

(11) **EP 2 383 001 B1**
(45) Date of publication and mention of the grant of the patent: **16.01.2013**
(21) Application number: 10161451.9
(22) Date of filing: 29.04.2010
(51) Int. Cl.: A61M 1/16, A61M 1/36

(54) **Oxygenator with integrated arterial filter**
Oxygenator mit integriertem Arterienfilter
Oxygénateur avec filtre artériel intégré

(43) Date of publication of application: 02.11.2011
(73) Proprietor: Sorin Group Italia S.r.l., 41037 Mirandola (MO) (IT)
(72) Inventor: Reggiani, Stefano, 41036, Medolla (Modena) (IT); Silvestri, Claudio, 41037, Quarantoli Mirandola (Modena) (IT); Giri, Alberto, 41037, Mirandola (Modena) (IT)
(74) Representative: Bosotti, Luciano

(56) References cited:
- WO-A2-97/16213
- US-A- 5 578 267
- US-A1- 2004 175 292

## Description

### TECHNICAL FIELD

The disclosure pertains generally to arterial filters used in blood perfusion systems.

### BACKGROUND

Blood perfusion entails encouraging blood through the vessels of the body. For such purposes, blood perfusion systems typically entail the use of one or more pumps in an extracorporeal circuit that is interconnected with the vascular system of a patient. Cardiopulmonary bypass surgery typically requires a perfusion system that provides for the temporary cessation of the heart to create a still operating field by replacing the function of the heart and lungs. Such isolation allows for the surgical correction of vascular stenosis, valvular disorders, and congenital heart defects. In perfusion systems used for cardiopulmonary bypass surgery, an extracorporeal blood circuit is established that includes at least one pump and an oxygenation device to replace the functions of the heart and lungs.

More specifically, in cardiopulmonary bypass procedures oxygen-poor blood, i.e., venous blood, is gravity-drained or vacuum suctioned from a large vein entering the heart or other veins in the body (e.g., femoral) and is transferred through a venous line in the extracorporeal circuit. The venous blood is pumped to an oxygenator that provides for oxygen transfer to the blood. Oxygen may be introduced into the blood by transfer across a membrane or, less frequently, by bubbling oxygen through the blood. Concurrently, carbon dioxide is removed across the membrane. The oxygenated blood is filtered and then returned through an arterial line to the aorta, femoral, or other artery.

Often, an arterial filter is added to the extracorporeal circuit, after the oxygenator, as last barrier before the patient, so as to block any solid or gaseous emboli and prevent any such emboli from entering into the aorta of the patient. Recently, arterial filters integrated in the oxygenator have been developed, allowing the reduction of the priming volume of the circuit and decreasing the global haemodilution of the patient. Specifically, the invention relates to an a oxygenator according to the preamble of clims 1, which is known e.g. from, WO 97/16123 A2. Also US 2004/175292 A1 and US 5 578 267 are of interest for the invention.

### SUMMARY

The object of the invention is an oxygenator having the features recited in claim 1.

According to an embodiment of the present invention, an oxygenator includes an oxygenator housing having a blood inlet and a blood outlet. The oxygenator housing defines an oxygenator volume. An annular filter housing defining an interior filter volume is disposed about the oxygenator housing. A filter is disposed within the annular filter housing, dividing the interior filter volume into a first chamber that is in fluid communication with the oxygenator volume and a second chamber that is fluid communication with the blood outlet. A first purge port is disposed within a wall forming the annular filter housing and is in fluid communication with the first chamber. A second purge port is disposed within the wall forming the annular filter housing and is in fluid communication with the second chamber.According to an embodiment of the present invention, such a blood processing apparatus includes an apparatus housing having a blood inlet and a blood outlet. The blood inlet may extend into an interior of the apparatus housing. A heat exchanger is in fluid communication with the blood inlet and is disposed about the blood inlet. A gas exchanger is disposed about the heat exchanger such that an inner surface of the gas exchanger is positioned to receive blood exiting an outer surface of the heat exchanger, an annular space being defined between an outer surface of the gas exchanger and an interior surface of the apparatus housing such that blood exiting the outer surface of the gas exchanger can collect in the annular space. An annular filter housing is arranged concentrically about the apparatus housing. A filter is arranged within the annular filter housing, forming a first annular chamber between the cylindrical filter and the apparatus housing and a second annular chamber between the cylindrical filter and the annular filter housing. An elongate opening is formed within the annular filter housing such that blood collecting in the annular space can pass into the first annular chamber. A first purge port is in communication with the first annular chamber and a second purge port is in communication with the second annular chamber.

According to another embodiment of the present invention, an integrated blood processing apparatus includes a housing having a blood inlet and a blood outlet, the blood inlet extending into an interior of the housing. A heat exchanger is disposed about the blood inlet and is in fluid communication with the blood inlet. An oxygenator is disposed about the heat exchanger and is in fluid communication with the heat exchanger. A filter housing defining an interior volume is secured to the housing. A filter is disposed within the filter housing, dividing the interior volume into a first chamber that is in fluid communication with the oxygenator and a second chamber that is in fluid communication with the blood outlet. A first purge port is in fluid communication with the first chamber and a second purge port is in fluid communication with the second chamber.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

Figure 1 is a schematic illustration of a blood processing apparatus including an integrated arterial filter in accordance with an embodiment of the invention.

Figure 2 is a cross-sectional illustration of the blood processing apparatus of Figure 1.

Figure 3 is an illustrative view of a filter deployed within the blood processing apparatus of Figure 1.

Figure 4 is an illustrative view of another filter deployed within the blood processing apparatus of Figure 1.

Figure 5 is a schematic illustration of a blood processing apparatus including an integrated arterial filter in accordance with an embodiment of the invention.

Figure 6 is a schematic cross-sectional illustration of the blood processing apparatus of Figure 5.

Figure 7 is an illustrative view of a filter deployed within the blood processing apparatus of Figure 5.

Figure 8 is a schematic illustration of a blood processing apparatus including an integrated arterial filter in accordance with an embodiment of the invention.

### DETAILED DESCRIPTION

The disclosure pertains to a blood processing apparatus that combines, in a single structure, a heat exchanger, a gas exchanger or oxygenator and an arterial filter. In some embodiments, the term oxygenator may be used to refer to a structure that combines a heat exchanger, a gas exchanger and an arterial filter in a unitary device. In some embodiments, an oxygenator may be used in an extracorporeal blood circuit. An extracorporeal blood circuit, such as may be used in a bypass procedure, may include several different elements such as a heart-lung machine, a blood reservoir, as well as an oxygenator.

In some embodiments, by combining the arterial filter with the oxygenator, the tubing set used to create the extracorporeal blood circuit may be reduced in complexity or number of parts and thus may simplify the extracorporeal blood circuit. In some embodiments, this will reduce the priming volume of the extracorporeal blood circuit.

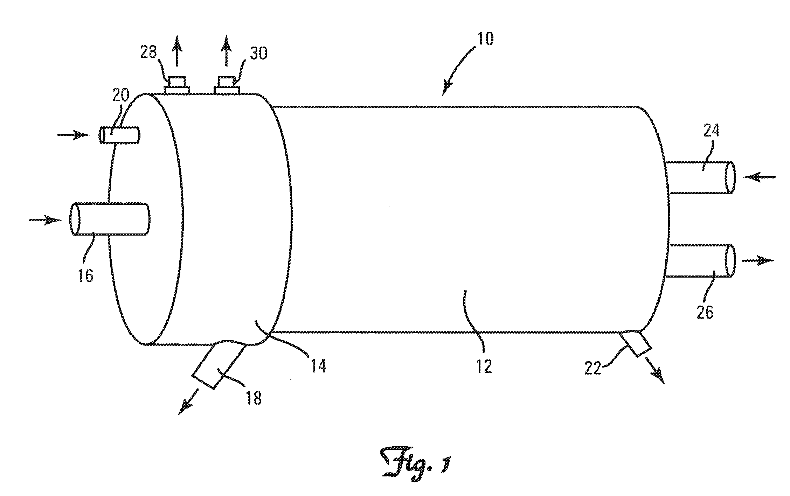

Figure 1 is a schematic illustration of a blood processing apparatus or oxygenator 10. While the internal components are not visible in this illustration, the oxygenator 10 may include one or more of a heat exchanger, a gas exchanger and an arterial filter. According to some embodiments, each of the heat exchanger, gas exchanger and arterial filter are integrated into a single structure that forms an oxygenator housing. The oxygenator 10 includes a device compartment or housing 12 and an arterial filter compartment or housing 14. In some embodiments, the arterial filter housing 14 may be integrally molded or otherwise structurally integrated with the device housing 12. In some cases, the arterial filter housing 14 may be separately formed and then secured or otherwise coupled to the device housing 12. According to various embodiments the heat exchanger, the gas exchanger, and the arterial filter housing 14 may have a cross-section shaped generally as a circle or as a parallelogram (e.g., a square or rectangle). Each of the heat exchanger, the gas exchanger and the arterial filter housing 14 may have generally the same sectional shape or each may have a different sectional shape.

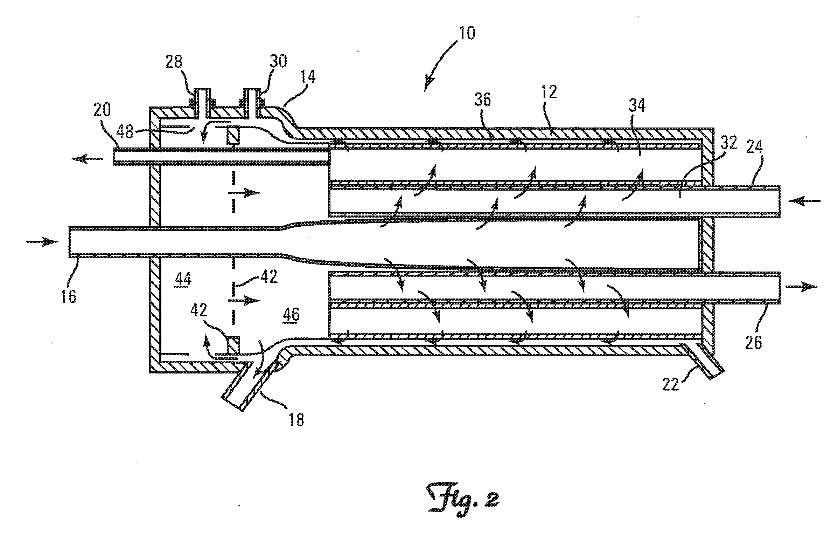

In some embodiments, a blood inlet 16 extends through the arterial filter housing 14 and into the device housing 12. A blood outlet 18 exits the arterial filter housing 14. As noted, in some embodiments the oxygenator 10 includes a gas exchanger and thus may include a gas inlet 20 and a gas outlet 22. In some embodiments, the oxygenator 10 includes a heat exchanger and thus may include a heating fluid inlet 24 and a heating fluid outlet 26. As will be explained in greater detail with respect to Figure 2, the oxygenator 10 includes a first purge port 28 and a second purge port 30. It is to be understood that the positions of the inlets, outlets and purge ports are merely illustrative, as other arrangements and configurations are contemplated. The purge ports may include a valve or a threaded cap. The purge ports operate to permit gases (e.g., air bubbles) that exit the blood to be vented or aspirated and removed from the oxygenator.

Figure 2 is a cross-sectional view of the oxygenator 10, illustrating internal components and an example of how blood can flow through the oxygenator 10. The oxygenator 10 includes a heat exchanger 32 and a gas exchanger 34. In some embodiments, the heat exchanger 32 includes a number of hollow fibers through which a heating fluid such as water can flow. The blood may flow around and past the hollow fibers and thus be suitably heated. In some embodiments, the hollow fibers may be polymeric. In some cases, metallic fibers may be used within the heat exchanger 32. According to other embodiments, the heat exchanger 32 includes a metal bellows or other structure comprising a substantial surface area (e.g., fins) for facilitating heat transfer with the blood.

In some embodiments the gas exchanger 34 may include a number of hollow fibers through which a gas such as oxygen may flow. The blood may flow around and past the hollow fibers. Due to concentration gradients, oxygen may diffuse through the hollow fibers into the blood while carbon dioxide may diffuse into the hollow fibers and out of the blood.

The oxygenator 10, according to some embodiments, includes an annular space 36 into which blood may flow as the blood exits the gas exchanger 34. As illustrated, the annular space 36 may extend into the arterial filter housing 14. According to exemplary embodiments, the annular space 16 may be generally circular or generally rectangular. The arterial filter housing 14 includes a filter 38. In some embodiments, the filter 38 includes an annular frame 40 and a net or mesh 42 spanning the annular frame 40. In some embodiments, the filter 38 may be considered as dividing a volume within the arterial filter housing 14 into a first chamber 44 and a second chamber 46. In various embodiments, the annular frame 40 and the net or mesh 42 are disposed concentrically with respect to the filter housing 14. In other embodiments the annular frame 40 and the mesh 42 are disposed about the housing 14 in a non-concentric manner. According to exemplary embodiments, the internal (i.e., priming) volume of the arterial filter housing 14 is between about 80 and about 110 mL. According to other embodiments, the priming volume is between about 90 and about 100 mL.

An opening 48 that may extend circumferentially up to about 360 degrees provides fluid communication between the annular space 36 and the first chamber 44. While blood is in the first chamber 44, any air bubbles that are present within the blood may be vented through the first purge port 28. Blood may pass through the filter 38 and into the second chamber 46. Any bubbles remaining in the blood, or caused by passage through the filter 38, may be vented through the second purge port 38. Blood may then exit the oxygenator 10 through the blood outlet 18. The presence of the first purge port 28 in the first chamber 44 and the second purge port 38 in the second chamber 46, according to various embodiments, will improve the priming speed due to the fact that bubbles present in the blood have both a first and a second opportunity to exit through a purge port. Moreover, in these embodiments, the efficacy of the bubble or gas removal is improved, again due to the fact that bubbles present in the blood have both a first and a second opportunity to exit through a purge port.

In some embodiments, the blood flow may be altered somewhat. For example, in some cases, the opening 48 may be positioned to provide fluid communication between the annular space 36 and the second chamber 46 while the blood outlet 18 is positioned in fluid communication with the first chamber 44. In some embodiments, the annular space 36 may empty directly into the second chamber 46, and may not extend into the first chamber 44.

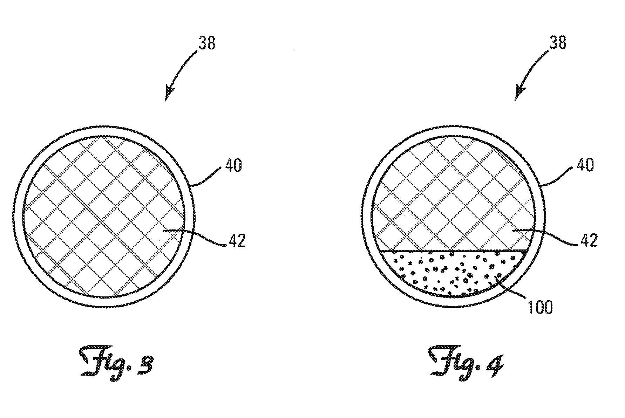

Figure 3 is a view of the filter 38, illustrating the frame 40 and the net or mesh 42. Figure 4 shows an embodiment of the filter 38 including a blocking plate 100. In some embodiments, the blocking plate 100 may be sized, shaped and positioned near the blood outlet 18 to limit preferential blood flow on the lower portion of the oxygenator 10. According to various embodiments, the filter 38 may have a cross-sectional shape that is circular, rectangular, or any other shape.

In some embodiments, the net or mesh 42 may have a mesh size that is the range of about 20 to about 200 microns. In some cases, the net or mesh 42 may have a mesh size of about 120 microns. In some instances, the net or mesh 42 may have a mesh size of from about 38-40 microns, and may be formed of a polymeric material such as polyester or polypropylene. In some cases, the net 42 may be coated with a biocompatible material. The blocking plate 100 may be formed of any suitable material. In some embodiments, the blocking plate 100 may be integrally formed with the frame 40. According to various exemplary embodiments, the net or mesh 42 has a surface area of between about 70 and about 90 square centimeters. According to other exemplary embodiments, the net or mesh 42 has a surface are of between about 75 and about 80 square centimeters.

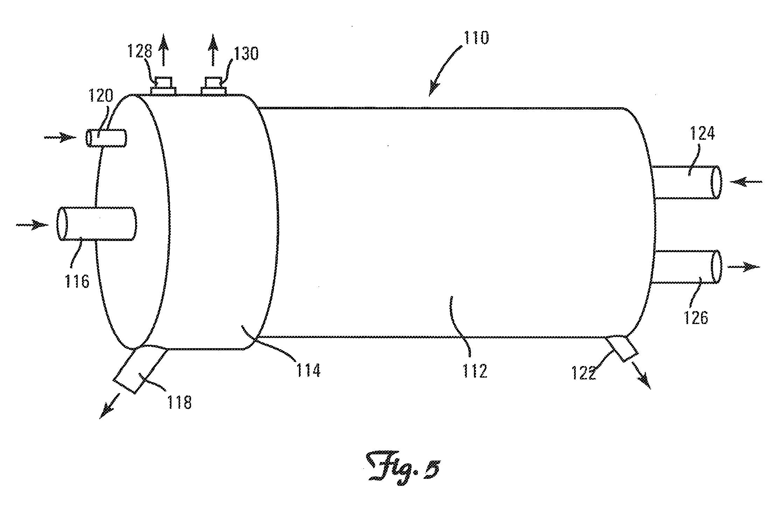

Figure 5 is a schematic illustration of a blood processing apparatus or oxygenator 110. While the internal components are not visible in this illustration, the oxygenator 110 may include one or more of a heat exchanger, a gas exchanger and an arterial filter. The oxygenator 110 includes a device housing 112 and an arterial filter housing 114. In some embodiments, the arterial filter housing 114 may be integrally molded or otherwise formed with the device housing 112. In some cases, the arterial filter housing 114 may be separately formed and then secured to the device housing 112.

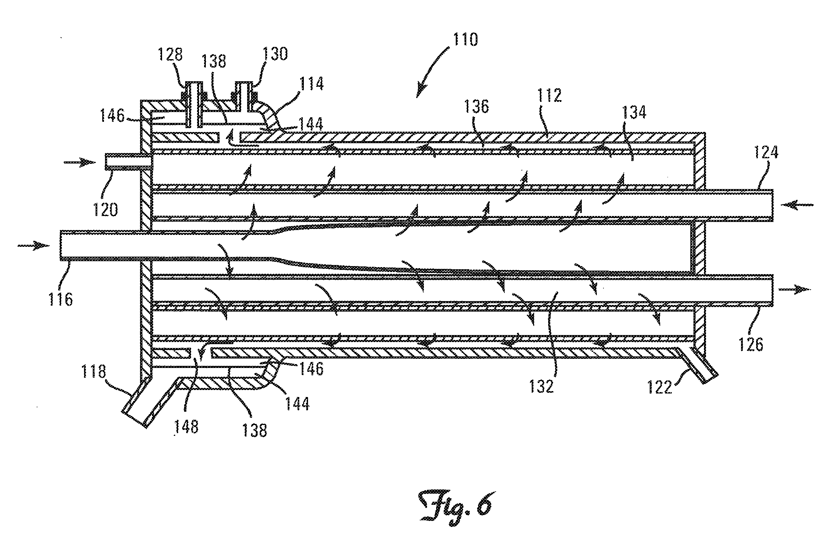

In some embodiments, a blood inlet 116 extends through the arterial filter housing 114 and into the device housing 112. A blood outlet 118 exits the arterial filter housing 114. As noted, in some embodiments the oxygenator 110 includes a gas exchanger and thus may include a gas inlet 120 and a gas outlet 122. In some embodiments, the oxygenator 110 includes a heat exchanger and thus may include a heating fluid inlet 124 and a heating fluid outlet 126. As will be explained in greater detail with respect to Figure 6, the oxygenator 110 includes a first purge port 128 and a second purge port 130. It is to be understood that the positions of the inlets, outlets and purge ports are merely illustrative, as other arrangements and configurations are contemplated.

Figure 6 is a cross-sectional view of the oxygenator 110, illustrating internal components and an example of how blood can flow through the oxygenator 110. The oxygenator 110 includes a heat exchanger 132 and a gas exchanger 134. In some embodiments, the heat exchanger 132 includes a number of hollow polymeric or metallic fibers through which a heating fluid such as water can flow. The blood may flow around and past the hollow fibers and thus be suitably heated. In some embodiments the gas exchanger 134 may include a number of hollow fibers through which a gas such as oxygen may flow. The blood may flow around and past the hollow fibers. Due to concentration gradients, oxygen may diffuse through the hollow fibers into the blood while carbon dioxide may diffuse into the hollow fibers and out of the blood.

As shown in Figure 6, the gas exchanger is configured such that blood flows radially across the gas exchanger 134. In these embodiments, the oxygenator 110 includes an annular space 136 into which blood may flow as the blood exits the gas exchanger 134. According to various embodiments, the annular space 136 may be either open or it may be partially or completely filled with hollow fibers. As illustrated, the arterial filter housing 114 may extend over a portion of the annular space 136. According to other embodiments, the gas exchanger 134, the heat exchanger 132, or both may be configured such that blood is directed in a longitudinal flow path. In various exemplary embodiments where the gas exchanger 134 is configured such that blood flows in a longitudinal path, the annular space 136 is omitted. In these embodiments, the blood flows out of the gas exchanger 134 near an end and flows directly into the arterial filter housing 114. In some embodiments, the opening between the gas exchanger 134 and the arterial filter housing 114 is blocked or occluded at the radial location corresponding to the blood outlet 18 of the arterial filter housing 14, to minimize or prevent direct flow from the gas exchanger 134 into the blood outlet 18.

A filter 138 may be disposed within the arterial filter housing 114. In some instances, as illustrated, the filter 138 divides the space within the annular filter housing 114 into a first chamber 144 and a second chamber 146. An opening 148 that may extend circumferentially up to about 360 degrees provides fluid communication between the annular space 136 and the first chamber 144. While blood is in the first chamber 144, any air bubbles that are present within the blood may be vented through the first purge port 128. Blood may pass through the filter 138 and into the second chamber 146. Any bubbles remaining in the blood, or caused by passage through the filter 138, may be vented through the second purge port 138. Blood may then exit the oxygenator 110 through the blood outlet 118.

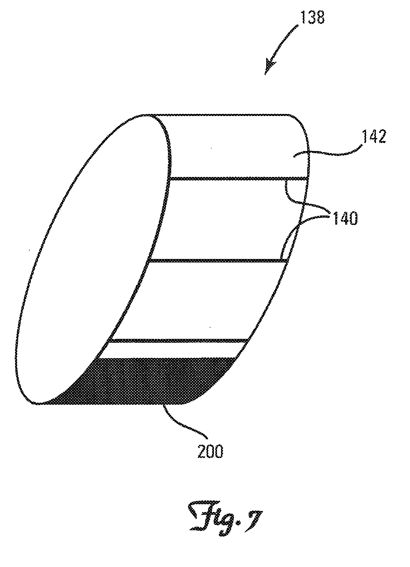

Figure 7 is a view of the filter 138. In some embodiments, the filter 138 is a cylindrical filter that includes one or more reinforcements 140 and a cylindrical net or mesh 142. In some embodiments, the one or more reinforcements 140 may be molded into the cylindrical net or mesh 142. In some cases, the one or more reinforcements 140 may be adhesively secured to the cylindrical net or mesh 142. In some embodiments, the one or more reinforcements 140 may extend cylindrically about the filter 138. In some instances, the one or more reinforcements 140 may run across the filter 138.

In some embodiments, the net or mesh 142 may have a mesh size that is the range of about 20 to about 200 microns. In some cases, the net or mesh 142 may have a mesh size of about 120 microns. In some instances, the net or mesh 142 may have a mesh size of about 40 microns, and may be formed of a polymeric material such as polyester or polypropylene. In some cases, the net 142 may be coated with a biocompatible material.

In some embodiments, the net or mesh 142 may include a blocking region or plate 200 that is sized, shaped and positioned near the blood outlet 118 to limit preferential blood flow on the lower portion of the oxygenator 110. The blocking plate 200 may be formed of any suitable material. In some embodiments, the blocking plate 200 may be molded or otherwise formed within the net or mesh 142.

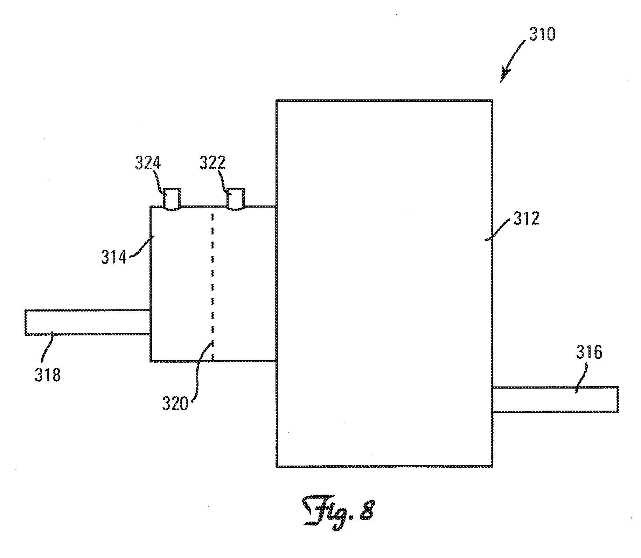

Figure 8 is a schematic illustration of a blood processing apparatus or oxygenator 310. While the internal components are not visible in this illustration, the oxygenator 310 may include one or more of a heat exchanger, a gas exchanger and an arterial filter. The oxygenator includes a device housing 312 and an arterial filter housing 314. In the illustrated embodiment, the arterial filter housing 314 is integrated into an end or side face of the device housing 312 and is configured such that blood exiting the device housing 312 enters the arterial filter housing 314. The device housing 312 includes a blood inlet 316 while the arterial filter housing 314 includes a blood outlet 318.

In some embodiments, as illustrated, the arterial filter housing 314 includes a net filter 320, a first purge port 322 and a second purge port 324. The first purge port 322 may be in fluid communication with an interior of the arterial filter housing 314 at a position upstream of the net filter 320 while the second purge port 324 may be in fluid communication with an interior of the arterial filter housing 314 at a position downstream of the net filter 320. As described in more detail above, this configuration allows an improvement and priming speed and efficacy, while also reducing the overall priming volume.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the above described features.

## Claims

1. An oxygenator comprising:
an oxygenator housing (12, 112, 312) having a blood inlet (16, 116, 316) and a blood outlet (18, 118, 318), the oxygenator housing defining an oxygenator volume;
a filter compartment structurally integrated into the oxygenator housing, the filter compartment defining an interior volume;
a filter (38, 138, 320) disposed within the filter compartment, the filter dividing the interior volume into a first chamber (44, 144) in fluid communication with the oxygenator volume and a second chamber (46, 146) in fluid communication with the blood outlet;
**characterized in that** it comprises:
- a first purge port (28, 128, 322) disposed within a wall forming the filter compartment, the first purge port in fluid communication with the first chamber; and
a second purge port (30, 130, 324) disposed within the wall forming the filter housing, the second purge port in fluid communication with the second chamber (46, 146).

2. The oxygenator of claim 1, wherein the filter (138) comprises a cylindrical net (142) including annular reinforcements (140).

3. The oxygenator of claim 1 or claim 2, wherein the filter (38) comprises:
a plate disposed across a lower portion of the filter, and/or a 40 micron mesh

4. The oxygenator of claim 1, comprising:
said blood inlet (16, 116) extending into an interior of the housing;
a heat exchanger (32, 132) disposed about the blood inlet, the heat exchanger being in fluid communication with the blood inlet;
an oxygenator (34, 134) disposed about the heat exchanger, the oxygenator being in fluid communication with the heat exchanger;
an annular filter housing (14, 114) disposed about the housing, the annular filter housing defining an interior volume;
a filter (38, 138) disposed within the annular filter housing, the filter dividing the interior volume into a first chamber in fluid communication with the oxygenator and a second chamber in fluid communication with the blood outlet;
said first purge port (28, 128) in fluid communication with the first chamber; and
said second purge port (30, 130) in fluid communication with the second chamber.

5. The oxygenator of claim 4, further comprising:
a heat exchanger fluid inlet (24, 124) and a heat exchanger fluid outlet (26, 126), both in fluid communication with the heat exchanger (32, 132), and/or
a gas inlet (20, 120) and a gas outlet (22, 122), both in fluid communication with the gas exchanger (34, 134).

6. The oxygenator of claim 4 or claim 5, wherein the filter (38, 138, 320) comprises a polymeric net, preferably comprising polyester or polypropylene.

7. The oxygenator of any of claims 4 to 6, wherein blood that enters the blood inlet passes through the heat exchanger (32, 132), the oxygenator (43, 134) and the filter (38, 138) before exiting the blood processing apparatus through the blood outlet.

8. The oxygenator of claim 1, comprising:
said blood inlet (16, 116) extending into an interior of the housing (12, 112);
a heat exchanger (32, 132) in fluid communication with the blood inlet (16, 116) and disposed about the blood inlet (16, 116);
a gas exchanger (34, 134) disposed about the heat exchanger (32, 132) such that an inner surface of the gas exchanger is positioned to receive blood exiting an outer surface of the heat exchanger, an annular space (36, 136) being defined between an outer surface of the gas exchanger and an interior surface of the apparatus housing such that blood exiting the outer surface of the gas exchanger can collect in the annular space;
an annular filter housing (14, 114) coupled concentrically about the apparatus housing;
said filter (38, 138) arranged within the annular filter housing, forming a first annular chamber (44, 144) between the filter and the apparatus housing and a second annular chamber (46, 146) between the filter and the annular filter housing;
an elongate opening (48, 148) formed within the annular filter housing such that blood collecting in the annular space can pass into the first annular chamber;
said first purge port (28, 128) in communication with the first annular chamber; and
said second purge port (30, 130) in communication with the second annular chamber.

9. The oxygenator of claim 8, wherein bubbles within the blood can exit through the first purge port (28, 128) and/or the second purge port (30, 130).

10. The oxygenator of claim 8 or claim 9, wherein the heat exchanger (32, 132) is configured to permit water flow therethrough in order to heat the blood passing through the heat exchanger.

11. The oxygenator of any of claims 8 to 10, wherein the gas exchanger (34, 134) is configured to permit gas to flow therethrough in order to add oxygen and remove carbon dioxide from the blood passing through the gas exchanger.

12. The oxygenator of any of the previous claims 8 to 11, wherein the heat exchanger (32, 132) comprises a cylindrical heat exchanger.

13. The oxygenator of any of the previous claims 8 to 12, wherein the gas exchanger (34, 134) comprises a cylindrical gas exchanger.

14. The oxygenator of any of the previous claims 8 to 13, wherein the blood inlet (16, 116) extends axially through the annular filter housing (14, 114).

15. The oxygenator of any of the previous claims 8 to 14, wherein the first purge port (28, 128) extends through the cylindrical filter such that the first purge port is in fluid communication with the first annular chamber.

## Patentansprüche

1. Ein Oxygenator mit:
einem Oxygenatorgehäuse (12, 112, 312) mit einem Bluteinlass (16, 116, 316) und einem Blutauslass (18, 118, 318), wobei das Oxygenatorgehäuse ein Oxygenatorvolumen definiert;
einem in dem Oxygenatorgehäuse strukturell integrierten Filterraum, der ein Innenvolumen definiert;
einem in dem Filterraum angeordneten Filter (38, 138, 320), wobei der Filter das Innenvolumen in eine erste Kammer (44, 144), welche in Fluidverbindung mit dem Oxygenatorvolumen steht, und in eine zweite Kammer (46, 146) aufteilt, welche in Fluidverbindung mit dem Blutauslass steht;
**dadurch gekennzeichnet, dass** der Oxygenator umfasst:
- einen ersten Spülanschluss (28, 128, 322), der innerhalb einer den Filterraum bildenden Wand angeordnet ist, wobei der erste Spülanschluss in Fluidverbindung mit der ersten Kammer steht; und
- einen zweiten Spülanschluss (30, 130, 324), der innerhalb der das Filtergehäuse bildenden Wand angeordnet ist, wobei der zweite Spülanschluss in Fluidverbindung mit der zweiten Kammer (46, 146) steht.

2. Oxygenator nach Anspruch 1, wobei der Filter (138) ein zylindrisches Netz (142) mit ringförmigen Verstärkungen (140) umfasst.

3. Oxygenator nach Anspruch 1 oder 2, wobei der Filter (38)
eine über einem unteren Bereich des Filters angeordnete Platte und/oder eine Maschenweite von 40 Mikrometer aufweist.

4. Oxygenator nach Anspruch 1, umfassend:
den Bluteinlass (16, 116), der sich in einen Innenraum des Gehäuses erstreckt;
einen Wärmetauscher (32, 132), der um den Bluteinlass herum angeordnet ist, wobei der Wärmetauscher in Fluidverbindung mit dem Bluteinlass steht;
einen Oxygenator (34, 134), der um den Wärmetauscher herum angeordnet ist, wobei der Oxygenator in Fluidverbindung mit dem Wärmetauscher steht;
ein ringförmiges Filtergehäuse (14, 114), das um das Gehäuse herum angeordnet ist, wobei das ringförmige Filtergehäuse ein Innenvolumen definiert;
einen Filter (38, 138), der innerhalb des ringförmigen Filtergehäuses angeordnet ist, wobei der Filter das Innenvolumen in eine erste Kammer, die in Fluidverbindung mit dem Oxygenator steht, und in eine zweite Kammer aufteilt, die in Fluidverbindung mit dem Blutauslass steht;
den ersten Spülanschluss (28, 128), der in Fluidverbindung mit der ersten Kammer steht; und
den zweiten Spülanschluss (30, 130), der in Fluidverbindung mit der zweiten Kammer steht.

5. Oxygenator nach Anspruch 4, weiterhin umfassend:
einen Wärmetauscherfluideinlass (24, 124) und einen Wärmetauscherfluidauslass (26, 126), die beide in Fluidverbindung mit dem Wärmetauscher (32, 132) stehen, und/oder
einen Gaseinlass (20, 120) und einen Gasauslass (22, 122), die beide in Fluidverbindung mit dem Gastauscher (34, 134) stehen.

6. Oxygenator nach Anspruch 4 oder 5, wobei der Filter (38, 138, 320) ein Polymernetz aufweist, das bevorzugt Polyester oder Polypropylen aufweist.

7. Oxygenator nach einem der Ansprüche 4 bis 6, wobei durch den Bluteinlass einströmendes Blut durch den Wärmetauscher (32, 132), den Oxygenator (43, 134) und den Filter (38, 138) strömt, bevor es die Blutaufbereitungsvorrichtung durch den Blutauslass verlässt.

8. Oxygenator nach Anspruch 1, umfassend:
den Bluteinlass (16, 116), der sich in einen Innenraum des Gehäuses (12, 112) erstreckt;
einen Wärmetauscher (32, 132), der in Fluidverbindung mit dem Bluteinlass (16, 116) steht und um den Bluteinlass (16, 116) herum angeordnet ist;
einen Gastauscher (34, 134), der um den Wärmetauscher (32, 132) herum derart angeordnet ist, dass eine Innenfläche des Gastauschers positioniert ist, um Blut aufzunehmen, das eine Außenfläche des Wärmetauschers verlässt, wobei ein ringförmiger Raum (36, 136) zwischen einer Außenfläche des Gastauschers und einer Innenfläche des Vorrichtungsgehäuses derart definiert ist, dass das die Außenfläche des Gastauschers verlassende Blut in dem ringförmigen Raum gesammelt werden kann;
ein ringförmiges Filtergehäuse (14, 114), welches konzentrisch um das Vorrichtungsgehäuse gekoppelt ist;
den Filter (38, 138), der innerhalb des ringförmigen Filtergehäuses angeordnet ist, wobei der Filter eine erste ringförmige Kammer (44, 144) zwischen dem Filter und dem Vorrichtungsgehäuse und eine zweite ringförmige Kammer (46, 146) zwischen dem Filter und dem ringförmigen Filtergehäuse bildet;
eine längliche Öffnung (48, 148), die derart in dem ringförmigen Filtergehäuse angeordnet ist, dass das sich in dem ringförmigen Raum sammelnde Blut durch die erste ringförmige Kammer strömen kann;
den ersten Spülanschluss (28, 128), der in Verbindung mit der ersten ringförmigen Kammer steht; und
den zweiten Spülanschluss (30, 130), der in Verbindung mit der zweiten ringförmigen Kammer steht.

9. Oxygenator nach Anspruch 8, wobei sich in dem Blut befindliche Blasen durch den ersten Spülanschluss (28, 128) und/oder den zweiten Spülanschluss (30, 130) austreten können.

10. Oxygenator nach Anspruch 8 oder 9, wobei der Wärmetauscher (32, 132) derart ausgebildet ist, dass Wasser durch diesen strömen kann, um das durch den Wärmetauscher strömende Blut zu erwärmen.

11. Oxygenator nach einem der Ansprüche 8 bis 10, wobei der Gastauscher (34, 134) derart ausgebildet ist, dass Gas durch diesen strömen kann, um dem durch den Gastauscher strömenden Blut Sauerstoff zuzuführen und Kohlendioxid zu entziehen.

12. Oxygenator nach einem der vorherigen Ansprüche 8 bis 11, wobei der Wärmetauscher (32, 132) einen zylindrischen Wärmetauscher umfasst.

13. Oxygenator nach einem der Ansprüche 8 bis 12, wobei der Gastauscher (34, 134) einen zylindrischen Gastauscher umfasst.

14. Oxygenator nach einem der Ansprüche 8 bis 13, wobei sich der Bluteinlass (16, 116) axial durch das ringförmige Filtergehäuse (14, 114) erstreckt.

15. Oxygenator nach einem der Ansprüche 8 bis 14, wobei sich der erste Spülanschluss (28, 128) durch den zylindrischen Filter derart erstreckt, dass der erste Spülanschluss in Fluidverbindung mit der ersten ringförmigen Kammer steht.

## Revendications

1. Oxygénateur comprenant :
un logement d'oxygénateur (12, 112, 312) comprenant une entrée de sang (16, 116, 316) et une sortie de sang (18, 118, 318), le logement d'oxygénateur définissant un volume d'oxygénateur ;
un compartiment de filtre structurellement intégré dans le logement d'oxygénateur, le compartiment de filtre définissant un volume intérieur ;
un filtre (38, 138, 320) disposé à l'intérieur du compartiment de filtre, le filtre divisant le volume intérieur en une première chambre (44, 144) en communication de fluide avec le volume d'oxygénateur et une seconde chambre (46, 146) en communication de fluide avec la sortie de sang ;
**caractérisé en ce qu'**il comprend :
- un premier orifice de purge (28, 128, 322) disposé à l'intérieur d'une paroi formant le compartiment de filtre, le premier orifice de purge étant en communication de fluide avec la première chambre ; et
- un second orifice de purge (30, 130, 324) disposé à l'intérieur de la paroi formant le logement de filtre, le second orifice de purge étant en communication de fluide avec la seconde chambre (46, 146).

2. Oxygénateur selon la revendication 1, dans lequel le filtre (138) comprend un filet cylindrique (142) comprenant des renforcements annulaires (140).

3. Oxygénateur selon la revendication 1 ou 2, dans lequel le filtre (38) comprend :
une plaque disposée à travers une partie inférieure du filtre et/ou une maille de 40 microns.

4. Oxygénateur selon la revendication 1, comprenant :
ladite entrée de sang (16, 116) s'étendant dans un intérieur du logement ;
un échangeur de chaleur (32, 132) disposé autour de l'entrée de sang, l'échangeur de chaleur étant en communication de fluide avec l'entrée de sang ;
un oxygénateur (34, 134) disposé autour de l'échangeur de chaleur, l'oxygénateur étant en communication de fluide avec l'échangeur de chaleur ;
un logement de filtre annulaire (14, 114) disposé autour du logement, le logement de filtre annulaire définissant un volume intérieur ;
un filtre (38, 138) disposé à l'intérieur du logement de filtre annulaire, le filtre divisant le volume intérieur en une première chambre en communication de fluide avec l'oxygénateur et une seconde chambre en communication de fluide avec la sortie de sang ;
ledit premier orifice de purge (28, 128) en communication de fluide avec la première chambre ; et
ledit second orifice de purge (30, 130) en communication de fluide avec la seconde chambre.

5. Oxygénateur selon la revendication 4, comprenant en outre :
une entrée de fluide d'échangeur de chaleur (24, 124) et une sortie de fluide d'échangeur de chaleur (26, 126), toutes deux en communication de fluide avec l'échangeur de chaleur (32, 132), et/ou
une entrée de gaz (20, 120) et une sortie de gaz (22, 122), toutes deux en communication de fluide avec l'échangeur de gaz (34, 134).

6. Oxygénateur selon la revendication 4 ou 5, dans lequel le filtre (38, 138, 320) comprend un filet polymère, comprenant de préférence du polyester ou du polypropylène.

7. Oxygénateur selon l'une quelconque des revendications 4 à 6, dans lequel du sang qui pénètre par l'entrée de sang traverse l'échangeur de chaleur (32, 132), l'oxygénateur (43, 134) et le filtre (38, 138) avant de sortir de l'appareil de traitement du sang par la sortie de sang.

8. Oxygénateur selon la revendication 1, comprenant :
ladite entrée de sang (16, 116) s'étendant dans un intérieur du logement (12, 112) ;
un échangeur de chaleur (32, 132) en communication de fluide avec l'entrée de sang (16, 116) et disposé autour de l'entrée de sang (16, 116) ;
un échangeur de gaz (34, 134) disposé autour de l'échangeur de chaleur (32, 132) de telle sorte qu'une surface interne de l'échangeur de gaz est positionnée de manière à recevoir le sang sortant d'une surface externe de l'échangeur de chaleur, un espace annulaire (36, 136) étant défini entre une surface externe de l'échangeur de gaz et une surface intérieure du logement d'appareil de telle sorte que le sang sortant de la surface externe de l'échangeur de gaz peut s'accumuler dans l'espace annulaire ;
un logement de filtre annulaire (14, 114) couplé concentriquement autour du logement d'appareil ;
ledit filtre (38, 138) disposé à l'intérieur du logement de filtre annulaire, formant une première chambre annulaire (44, 144) entre le filtre et le logement d'appareil et une seconde chambre annulaire (46, 146) entre le filtre et le logement de filtre annulaire ;
une ouverture allongée (48, 148) formée à l'intérieur du logement de filtre annulaire de telle sorte que le sang s'accumulant dans l'espace annulaire puisse passer dans la première chambre annulaire ;
ledit premier orifice de purge (28, 128) en communication avec la première chambre annulaire ; et
ledit second orifice de purge (30, 130) en communication avec la seconde chambre annulaire.

9. Oxygénateur selon la revendication 8, dans lequel les bulles à l'intérieur du sang peuvent sortir à travers le premier orifice de purge (28, 128) et/ou le second orifice de purge (30, 130).

10. Oxygénateur selon la revendication 8 ou 9, dans lequel l'échangeur de chaleur (32, 132) est configuré pour permettre un écoulement d'eau à travers lui pour réchauffer le sang traversant l'échangeur de chaleur.

11. Oxygénateur selon l'une quelconque des revendications 8 à 10, dans lequel l'échangeur de gaz (34, 134) est configuré pour permettre au gaz de s'écouler à travers lui afin d'ajouter de l'oxygène et d'éliminer le dioxyde de carbone du sang traversant l'échangeur de chaleur.

12. Oxygénateur selon l'une quelconque des revendications précédentes 8 à 1l, dans lequel l'échangeur de chaleur (32, 132) comprend un échangeur de chaleur cylindrique.

13. Oxygénateur selon l'une quelconque des revendications 8 à 12 précédentes, dans lequel l'échangeur de gaz (34, 134) comprend un échangeur de gaz cylindrique.

14. Oxygénateur selon l'une quelconque des revendications 8 à 13 précédentes, dans lequel l'entrée de sang (16, 116) s'étend axialement à travers le logement de filtre annulaire (14, 114).

15. Oxygénateur selon l'une quelconque des revendications 8 à 14 précédentes, dans lequel le premier orifice de purge (28, 128) s'étend à travers le filtre cylindrique de telle sorte que le premier orifice de purge soit en communication de fluide avec la première chambre annulaire.
